(19) Europäisches Patentamt
European Patent Office
Office européen des brevets

(11) Veröffentlichungsnummer : **0 382 050 B1**

(12) # EUROPÄISCHE PATENTSCHRIFT

(45) Veröffentlichungstag der Patentschrift :
**28.07.93 Patentblatt 93/30**

(51) Int. Cl.$^5$ : **C07C 29/136,** C07C 31/20

(21) Anmeldenummer : **90101786.3**

(22) Anmeldetag : **30.01.90**

(54) **Verfahren zur Herstellung von Alkanolen.**

(30) Priorität : **04.02.89 DE 3903363**

(43) Veröffentlichungstag der Anmeldung :
**16.08.90 Patentblatt 90/33**

(45) Bekanntmachung des Hinweises auf die Patenterteilung :
**28.07.93 Patentblatt 93/30**

(84) Benannte Vertragsstaaten :
**BE CH DE FR GB IT LI NL**

(56) Entgegenhaltungen :
**EP-A- 0 304 696**
**DE-A- 2 321 101**
**US-A- 3 478 112**
**US-A- 3 848 003**
**US-A- 4 141 930**

(73) Patentinhaber : **BASF Aktiengesellschaft**
**Carl-Bosch-Strasse 38**
**W-6700 Ludwigshafen (DE)**

(72) Erfinder : **Weyer, Hans-Juergen, Dr.**
**Neckarpromenade 20**
**W-6800 Mannheim 1 (DE)**
Erfinder : **Fischer, Rolf, Dr.**
**Bergstrasse 98**
**W-6900 Heidelberg (DE)**
Erfinder : **Mueller, Franz-Josef, Dr.**
**Mueller-Thurgau-Weg 1**
**W-6706 Wachenheim (DE)**
Erfinder : **Zimmermann, Horst, Dr.**
**Ringstrasse 36**
**W-6900 Heidelberg (DE)**

EP 0 382 050 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf ein Verfahren zur Herstellung von Alkanolen, die mindestens zwei Hydroxylgruppen enthalten, durch katalytische Hydrierung von 5- und 6-gliedrigen Lactonen in Gegenwart von Kobaltkatalysatoren.

Für die Herstellung von Alkandiolen wie Butandiol-1,4, Pentandiol-1,5 und Hexandiol-1,6 sind zahlreiche Methoden bekamt. So lassen sich solche Diole z.B. durch Umsetzung von Acetylen mit Aldehyden und Hydrierung der zunächst erhaltenen Alkindiole herstellen. Weiterhin besteht die Möglichkeit, Dicarbonsäuren oder ihre Ester zu entsprechenden Diolen zu hydrieren.

Man hat auch schon die Hydrierung von Lactonen für die Herstellung von Alkandiolen herangezogen. So ist z.B. aus den Patentschriften GB 1 230 276, GB 1 344 557, GB 1 512 751 und US 4 652 685 bekannt, daß man Butyrolacton in der Gas- oder Flüssigphase an Katalysatoren aus Kupferoxid und Chromoxid zu 1,4-Butandiol hydrieren kann. Um vorteilhafte Ergebnisse zu erzielen, hydriert man bei Temperaturen von über 180°C und Drücken von über 80 bar. Die verwendeten Katalysatoren sind wegen ihres hohen Chromgehaltes nicht einfach zu entsorgen. In der GB-PS 1 314 126 wird die Hydrierung von γ-Butyrolacton an einem Katalysator beschrieben, der Nickel, Kobalt und Thorium enthält. Hierbei wirft sowohl die Handhabung als auch die Entsorgung des α-Strahlers Thorium 232 Probleme auf. Nach den Angaben der US-PS 4,301 077 hydriert man γ-Butyrolacton an einem Katalysator, der z.B. Nickel, Kobalt, Zink und Ruthenium enthält. Dabei erhält man 1,4-Butandiol z.B. bei halbem Umsatz nur mit einer Selektivität von 83 %.

EP-A 304 696 betrifft ein Verfahren zur Herstellung von 1,4-Butandiol und/oder Tetrahydrofuran durch die katalytische Hydrierung von Maleinsäureanhydrid, Bernsteinsäureanhydrid, Maleinsäure, Bernsteinsäure, Fumarsäure oder den Alkylestern dieser Säuren bei Temperaturen von 100 bis 350°C und Drücken von 50 bis 350 bar an einem Katalysator, der als katalytisch aktives Metall Kobalt und mindestens eines der Elemente Kupfer und Phosphor enthält. Gemäß Beispiel 7 dieser Schrift wird bei der Hydrierung eines Gemisches aus 15 % 1,4-Butandiol, 55 % Tetrahydrofuran, 25 % γ-Butyrolacton, 2 % Butanol und Propanol bei 260°C und bei einem Druck von 300 bar ein Produkt erhalten, das 93 % 1,4-Butandiol und THF, 1,5 % γ-Butyrolacton, 2,5 % Butanol und 0,4 % Propanol enthält.

Es bestand deshalb die Aufgabe, ein Verfahren zur Hydrierung von Lactonen zu Alkanolen zu finden, das diese Nachteile nicht aufweist.

Es wurde nun gefunden, daß man Alkanole der allgemeinen Formel

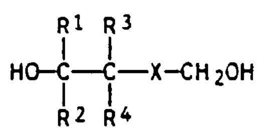

I,

in der X das Brückenglied

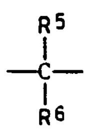

oder

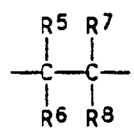

,

$R^1$ bis $R^8$ Wasserstoffatome, Hydroxylgruppen oder Alkyl-, Alkoxi-, oder Hydroxialkylreste mit 1 bis 4 C-Atomen bedeuten, und die Reste $R^1$, $R^3$, $R^5$ und $R^7$ auch für Cycloalkylreste mit 5 bis 7 C-Atomen stehen können, besonders vorteilhaft herstellen kann, wenn man Lactone der allgemeinen Formel

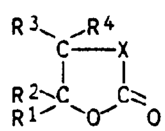

II,

in der X und die Reste R die obengenannte Bedeutung haben, bei Temperaturen von 100 bis 210°C und Drücken von 20 bis 350 bar in Gegenwart eines Katalysators hydriert, der Kobalt und mindestens eines der Elemente Mangan, Kupfer und Phosphor enthält.

Vorzugsweise stellt man nach dem neuen Verfahren Alkanole der allgemeinen Formel

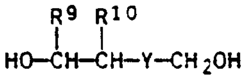

III,

in der Y das Brückenglied $-CHR^{11}-$ oder $-CHR^{11}-CHR^{12}-$ und $R^9$ bis $R^{12}$ Wasserstoffatome, Hydroxylgruppen oder Alkyl-, Alkoxy- oder Hydroxialkylreste mit 1 bis 4 C-Atomen bedeuten, aus den entsprechenden Lactonen der allgemeinen Formel

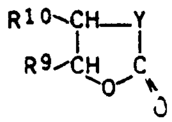

IV,

her.

In den Lactonen der Formeln II oder IV kommen als Alkyl-, Alkoxi- oder Hydroxialkylgruppen z.B. die folgenden Gruppen in Betracht: Methyl, Ethyl, Propyl, Butyl, Methoxi, Ethoxi, Hydroxiethyl, Hydroxipropyl oder Hydroxibutyl. Cycloalkylreste sind z.B. Cyclohexyl oder Cyclopentyl. Als Lactone der Formel II seien z.B. die folgenden Verbindungen genannt γ-Butyrolacton, δ-Valerolacton, 5-Methylbutyrolacton, 6-Methylvalerolacton, 5-Hydroxymethylbutyrolacton, 3-methylbutyrolacton, 5-(3-Hydroxipropyl)butyrolacton, 3-Hydroxibutyrolacton, 3-methoxibutyrolacton und 4-Hydroxi-5-methylbutyrolacton.

Die erfindungsgemäß zu verwendenden Kobaltkatalysatoren enthalten Kobalt und mindestens eines der Elemente Mangan, Kupfer und Phosphor. Bevorzugt enthalten die Katalysatoren neben Kobalt mindestens zwei der Elemente Mangan, Kupfer, Phosphor und Molybdän. Von besonderem technischem Interesse sind solche Katalysatoren, die neben Kobalt mindestens drei der Elemente Mangan, Kupfer, Phosphor, Molybdän und Natrium enthalten. Dabei können die Metalle in metallischer Form oder in Form ihrer Oxide nach Reduktion, z.B. mit Wasserstoff, eingesetzt werden. Phosphor liegt praktisch in Form von Phosphorsäure vor.

Geeignete Katalysatoren sind z. B. solche, deren katalytisch aktive Masse mindestens zu 40 Gew.-%, z.B. zu 40 bis 80 Gew.% aus Kobalt besteht und als weitere aktive Bestandteile bis zu 10 Gew.-%, z. B. 3 bis 7 Gew.-% Mangan, bis zu 10 Gew.-%, z. B. 0,1 bis 3 Gew.-% Phosphor und bis zu 1 Gew.-%, z. B. 0.01 bis 0.5 Gew.-% Natrium enthält. Besonders gute Ergebnisse werden z. B. auch mit solchen Katalysatoren dieser Art erzielt, deren katalytisch aktive Masse als weitere aktive Bestandteile bis zu 30 Gew.-%, z. B. 12 bis 20 Gew.-% Kupfer und bis zu 5 Gew.-%, z. B. 0,5 bis 5 Gew.% Molybdän enthalten.

Die Katalysatoren werden als Trägerkatalysatoren oder vorzugsweise als Substanzkatalysatoren eingesetzt. Trägerkatalysatoren werden auf an sich bekannte Weise, zweckmäßigerweise durch ein- oder mehrmaliges Imprägnieren des Trägermaterials mit einer wäßrigen Lösung der Metallsalze und der Phosphorverbindung hergestellt. Nach dem Imprägnieren und Trocknen wird die Masse wie an sich üblich, z.B. auf Temperaturen von 400 bis 600°C erhitzt, wobei die Metallsalze in die Metalloxide übergehen. Zur Herstellung der Substanzkatalysatoren verfährt man auf an sich bekannte Weise z.B. so, daß man ein Kobaltsalz, z.B. Kobaltnitrat, in wäßriger Lösung mit den wäßrigen Salzlösungen (z.B. ebenfalls den Nitraten) der anderen Metalle, mit Zunahme von Molybdän und gegebenenfalls mit Phosphorsäure mischt, die erhaltene wäßrige Lösung mittels Alkalicarbonatlösung bei einer Temperatur zwischen 40 und 70°C auf einen pH-Wert von wenigstens 8 bringt, die erhaltene Suspension zur Vervollständigung der Fällung auf einen pH-Wert unterhalb von 7,5 bringt, die ausgefällte Masse gewinnt, trocknet, bei 400 bis 600°C calciniert, die abgekühlte Masse gegebenenfalls nachwäscht, mit einer Lösung eines Salzes der Molybdänsäure tränkt und durch eine anschließende Säurebehandlung die Molybdänsäure auf der Masse fixiert. Die Katalysatormasse wird dann in an sich bekannter Weise geformt, getrocknet und reduziert, wobei die Mengen der Lösungen jeweils so gewählt werden, daß der Katalysator im calcinierten, nicht reduzierten Zustand die angegebene Zusammensetzung hat, wobei die Metalle in Form ihrer Oxide und Phosphor als Phosphorsäure vorliegen. Vor ihrem Einsatz werden die Katalysatoren mit Wasserstoff behandelt, wobei die Oxide größtenteils zu den Metallen reduziert werden.

Als Katalysatorträger sind z. B. Siliziumdioxid, Aluminiumdioxid, Titandioxid, Aktivkohle, Silikate oder Zeolithe geeignet. Wenn nötig können bei der Katalysatorherstellung Bindemittel verwendet werden.

Das Lacton kann in reiner Form oder in inerten Lösungsmitteln, wie Alkanen, Ethern oder Alkoholen eingesetzt werden. Geeignete Alkohole sind beispielsweise Ethanol, Propanol und Butanol. Als etherische Lösungsmittel können z. B. Dibutylether und Tetrahydrofuran verwendet werden. Vorteilhaft ist der Einsatz von Butanol als alkoholisches Lösungsmittel.

Man hydriert bei Temperaturen von 100 bis 210°C, vorzugsweise bei 100 bis 170°C und bei Drücken von 20 bis 350 bar, vorzugsweise bei 50 bis 150, insbesondere bei 50 bis 130 bar, wobei die Katalysatorbelastung je nach Temperatur und Druck 0.05 bis 5.0 kg/(1h) betragen kann. Die Hydrierung wird in der Flüssig- oder Gasphase durchgeführt. Man arbeitet diskontinuierlich oder kontinuierlich in Sumpf- oder Rieselfahrweise, wobei der Katalysator normalerweise fest angeordnet ist. Es ist aber auch möglich, einen Katalysator einzusetzen, der in dem Reaktionsgemisch suspendiert ist.

Nach dem erfindungsgemäßen Verfahren erhält man die Alkanole schon bei Temperaturen und Drücken, die im unteren Teil der oben angegebenen Temperatur- und Druckbereiche liegen, in guten Ausbeuten.

Die erfindungsgemäß dargestellten Alkandiole sind wertvolle Ausgangsprodukte z. B. für die Herstellung von Polyestern.

Die in den Beispielen genannten Prozente sind Gewichtsprozente

Beispiel 1, zum Vergleich

Die Hydrierung wurde in einem Rohrreaktor durchgeführt, der über einen außenliegenden Mantel mit Öl auf die erforderlichen Temperaturen aufgeheizt werden konnte. Der beheizte Innenraum des Rohrreaktors hatte einen Durchmesser von 10 mm und war 1430 mm lang. Das untere Drittel des Innenraums enthielt die Katalysatorschüttung. Das mittlere Drittel war mit Glasringen gefüllt. Das obere Drittel war leer. Gasförmiger und flüssiger Zulauf durchströmten den Rohrreaktor gemeinsam von oben nach unten. Den Reaktoraustrag kühlte man auf Raumtemperatur und trennte ihn in einen flüssigen und einen gasförmigen Strom.

Der verwendete Katalysator, von dem 62,3 g eingesetzt wurden, hatte die Zusammensetzung 66,8 % CoO (= 52,7 % Co), 19,1 % CuO (= 15,3 % Cu), 7,1 % $Mn_2O_3$ (= 5,1 % Mn), 3,3 % $MoO_3$ (= 2,2 % Mo), 3,5 Gew.% $H_3PO_4$ (= 1,1 % P) und 0,15 Gew.% $Na_2O$ (= 0,1 % Na). Der Katalysator hatte die äußere Form von Strängen mit dem Durchmesser 3 mm und der Höhe 2 mm.

Der Reaktor wurde zur Aktivierung des Katalysators zunächst mit Wasserstoff luftfrei gespült. Bei einem Gesamtdruck von 15 bar und einem Wasserstoffstrom von mindestens 500 l/h steigerte man die Temperatur in einem Zeitraum von 7 Stunden auf 310°C. Danach erhöhte man den Gesamtdruck auf 250 bar und den Wasserstoffstrom auf mindestens 100 l/h. Man hielt diese Bedingungen mindestens 72 Stunden lang aufrecht. Danach wurde mit der Hydrierung begonnen.

Bei einem Gesamtdruck von 60 bar und einer Temperatur von 208 °C wurden dem Reaktor stündlich 98 g gamma-Butyrolacton (1,14 mol) und 1212 Liter Wasserstoff (54,1 mol) zugeführt. Der stündliche flüssige Reaktoraustrag enthielt neben 51,6 g unumgesetztem gamma-Butyrolacton (0,60 mol) 35,1 g 1.4-Butandiol (0,39 mol), 0,15 g THF (0,002 mol), 0,6 g n-Butanol (0,01 mol), 0,4 g n-Propanol (0,006 mol) und 0,4 g Wasser (0,022 mol).

Beispiel 2

Eine Mischung aus 70 g 5-(3-Hydroxipropyl)butyrolacton und 70 g n-Butanol wurde in Gegenwart von 20 g des in Beispiel 1 verwendeten und mit Wasserstoff aktivierten Katalysators bei 200 bar und 200°C diskontinuierlich hydriert. Nach vollständigem Umsatz waren 83 % Heptantriol-1,4,7, 8 % 2-(3-Hydroxipropyl)-tetrahydrofuran und 9 % andere Produkte, wie Heptandiole und Hexandiol-1,4 entstanden. Durch Destillation wurden 48,2 g (67 Mol-%) Heptantriol-1,4,7 erhalten.

Beispiel 3

Eine Mischung aus 10 g 4-Hydroxi-5-methyl-γ-butyrolacton und 100 g n-Butanol wurde in Gegenwart von 20 g des in Beispiel 1 verwendeten und mit Wasserstoff aktivierten Katalysators bei 200 bar und 150°C im Autoklaven umgesetzt. Entsprechend der gaschromatographischen Analyse hatte sich das Edukt vollständig unter Bildung von Pentantriol-1,3,4 (4,3 %) und Pentandiol-1,4 (84,0 %) umgesetzt.

Beispiele 4 bis 8

Für die Beispiele 4 bis 8 wurden jeweils 30 g eines Lactons und 100 g n-Butanol zusammen mit 20 g des in Beispiel 1 verwendeten und mit Wasserstoff aktivierten Katalysators eingesetzt. Diese Mischung wurde in einem 300-ml-Autoklav bei 200 bar bis zur Druckkonstanz hydriert. Reaktionstemperatur, Art des verwendeten Lactons und Produktzusammensetzung laut gaschromatographischer Analyse sind Tabelle 1 zu entnehmen.

Tabelle

| Beispiel | Lacton der Formel II | Temperatur [°C] | Alkanol der Formel I**) [Mol%] | THF-Derivat**) [Mol%] |
|---|---|---|---|---|
| 4 | alle R = H | 125 | 98,0 | 0,1 |
| 5 | $R^1$ = $CH_3$ *) | 175 | 93,1 | 2,4 |
| 6 | $R^3$ = $CH_3$ *) | 200 | 54,3 | 27,3 |
| 7 | $R^5$ = $CH_3$ *) | 200 | 56,8 | 26,8 |
| 8 | $R^1$ = $CH_2OH$ *) | 150 | 90,7 | 4,8 |

*) alle nicht genannten R-Reste stehen für H

**) mit den entsprechenden Substituenten der eingesetzten Lactone

Beispiel 9, zum Vergleich

Der in Beispiel 1 beschriebene Versuch wurde bei 30 bar und 230°C in der Gaspase mit einer stündlichen Reaktorzuführung von 145 g γ-Butyrolacton (1,69 mol) und 686 l Wasserstoff (30,6 mol) durchgeführt. Der stündliche flüssige Reaktoraustrag enthielt neben 108,4 g unumgesetztem α-Butyrolacton (1,26 mol) 36,0 g Butandiol-1,4 (0,4 mol), 0,75 g THF (0,01 mol), 1,86 g Butanol (0,031 mol), 1,8 g Propanol (0,027 mol) und 1,7 g Wasser (0,086 mol).

## Patentansprüche

1. Verfahren zur Herstellung von Alkanolen der allgemeinen Formel

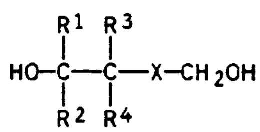 I,

in der X das Brückenglied

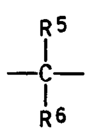 oder 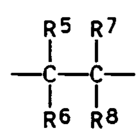 ,

$R^1$ bis $R^8$ Wasserstoffatome, Hydroxylgruppen oder Alkyl-, Alkoxi- oder Hydroxialkylreste mit 1 bis 4 C-Atomen bedeuten, und die Reste $R^1$, $R^3$, $R^5$ und $R^7$ auch für Cycloalkylreste mit 5 bis 7 C-Atomen stehen können, dadurch gekennzeichnet, daß man Lactone der allgemeinen Formel

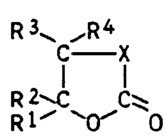 II,

in der X und die Reste R die obengenannte Bedeutung haben, bei Temperaturen von 100 bis 210°C und Drücken von 20 bis 350 bar in Gegenwart eines Katalysators hydriert, der Kobalt und mindestens eines

der Elemente Mangan, Kupfer und Phosphor enthält.

2. Verfahren nach Anspruch 1, dadurch gekennzeichnet, daß man einen Katalysator verwendet, der neben Kobalt mindestens zwei der Elemente Mangan, Kupfer, Phosphor und Molybdän enthält.

3. Verfahren nach Anspruch 1, dadurch gekennzeichnet, daß man einen Katalysator verwendet, der Kobalt und mindestens drei der Elemente Mangan, Kupfer, Phosphor, Molybdän und Natrium enthält.

4. Verfahren nach Anspruch 1, dadurch gekennzeichnet, daß die aktive Masse des Katalysators mindestens zu 40 Gew.% aus Kobalt besteht.

5. Verfahren nach Anspruch 3, dadurch gekennzeichnet, daß man einen Katalysator verwendet, dessen aktive Masse mindestens zu 40 Gew.% aus Kobalt besteht und als weitere aktive Bestandteile bis zu 10 Gew.% Mangan, bis zu 10 Gew.% Phosphor und bis zu 1 Gew.% Natrium enthält.

6. Verfahren nach Anspruch 3, dadurch gekennzeichnet, daß man einen Katalysator verwendet, dessen aktive Massen zu 40 bis 80 Gew.% aus Kobalt, 3 bis 7 Gew.% aus Mangan, 0,1 bis 3 Gew.% aus Phosphor und 0,01 bis 0,5 Gew.% aus Natrium besteht.

7. Verfahren nach Anspruch 1, dadurch gekennzeichnet, daß man einen Katalysator verwendet, dessen aktive Masse mindestens zu 40 Gew.% aus Cobalt besteht und als weitere aktive Bestandteile bis zu 10 Gew.% Mangan, bis zu 30 Gew.% Kupfer, bis zu 5 Gew.% Molybdän, bis zu 10 Gew.% Phosphor und bis zu 1 Gew.% Natrium enthält.

8. Verfahren nach Anspruch 3, dadurch gekennzeichnet, daß man einen Katalysator verwendet, dessen aktive Massen zu 40 bis 60 Gew.% aus Kobalt, 3 bis 7 Gew.% aus Mangan, 0,1 bis 3 Gew.% aus Phosphor, 12 bis 20 Gew.% aus Kupfer, 0,5 bis 5 Gew.% aus Molybdän und zu 0,01 bis 0,5 Gew.% aus Natrium besteht.

## Claims

1. A process for the preparation of an alkanol of the formula

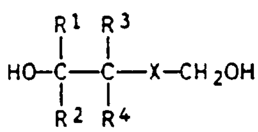

I

where X is a bridge member

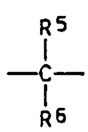

or

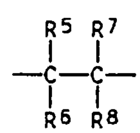

where $R^1$ to $R^8$ are each hydrogen, hydroxyl or an alkyl, alkoxy or hydroxyalkyl radical of 1 to 4 carbon atoms and $R^1$, $R^3$, $R^5$ and $R^7$ may furthermore be cycloalkyl of 5 to 7 carbon atoms, wherein a lactone of the formula

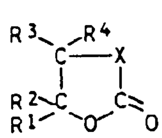

II

where X and the radicals R have the abovementioned meanings, is hydrogenated at from 100 to 210°C

and under from 20 to 350 bar in the presence of a catalyst which contains cobalt and one or more of the elements manganese, copper and phosphorus.

2. A process as claimed in claim 1, wherein the catalyst used contains cobalt as well as two or more of the elements manganese, copper, phosphorus and molybdenum.

3. A process as claimed in claim 1, wherein the catalyst used contains cobalt and three or more of the elements manganese, copper, phosphorus molybdenum and sodium.

4. A process as claimed in claim 1, wherein the active material of the catalyst consists of not less than 40% by weight of cobalt.

5. A process as claimed in claim 3, wherein a catalyst is used whose active material consists of not less than 40% by weight of cobalt and contains, as further active components, not more than 10% by weight of manganese, not more than 10% by weight of phosphorus and not more than 1% by weight of sodium.

6. A process as claimed in claim 3, wherein a catalyst is used whose active material consists of from 40 to 80% by weight of cobalt, from 3 to 7% by weight of manganese, from 0.1 to 3% by weight of phosphorus and from 0.01 to 0.5% by weight of sodium.

7. A process as claimed in claim 1, wherein a catalyst is used whose active material consists of not less than 40% by weight of cobalt and contains, as further active components, not more than 10% by weight of manganese, not more than 30% by weight of copper, not more than 5% by weight of molybdenum, not more than 10% by weight of phosphorus and not more than 1% by weight of sodium.

8. A process as claimed in claim 3, wherein a catalyst is used whose active material consists of from 40 to 60% by weight of cobalt, from 3 to 7% by weight of manganese, from 0.1 to 3% by weight of phosphorus, from 12 to 20% by weight of copper, from 0.5 to 5% by weight of molybdenum and from 0.01 to 0.5% by weight of sodium.

## Revendications

1. Procédé de préparation d'alcanols de formule générale

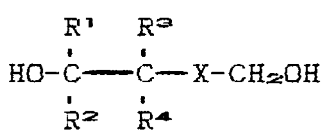

I

dans laquelle X représente le maillon de pontage

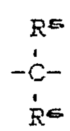

ou

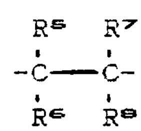

$R^1$ à $R^8$ représentent des atomes d'hydrogène, des groupements hydroxy ou des restes alkyle, alcoxy ou hydroxyalkyle à 1-4 atomes de carbone, et les restes $R^1$, $R^3$, $R^5$ et $R^7$ peuvent aussi être mis pour des restes cycloalkyle à 5-7 atomes de carbone, caractérisé en ce qu'on hydrogène des lactones de formule générale

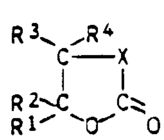

II,

dans laquelle X et les restes R ont les significations sus-indiquées, à des températures de 100 à 210°C et sous des pressions de 20 à 350 bar, en présence d'un catalyseur qui contient du cobalt et l'un au moins des éléments manganèse, cuivre et phosphore.

**2.** Procédé selon la revendication 1, caractérisé en ce qu'on utilise un catalyseur qui contient, outre du cobalt, au moins deux des éléments manganèse, cuivre, phosphore et molybdène.

**3.** Procédé selon la revendication 1, caractérisé en ce qu'on utilise un catalyseur qui contient du cobalt et au moins trois des éléments manganèse, cuivre, phosphore, molybdène et sodium.

**4.** Procédé selon la revendication 1, caractérisé en ce que la masse active du catalyseur se compose de cobalt pour 40% en poids au moins.

**5.** Procédé selon la revendication 3, caractérisé en ce qu'on utilise un catalyseur dont la masse active se compose de cobalt pour 40% en poids au moins et contient, comme autres contituants actifs, jusqu'à 10% en poids de manganèse, jusqu'à 10% en poids de phosphore et jusqu'à 1% en poids de sodium.

**6.** Procédé selon la revendication 3, caractérisé en ce qu'on utilise un catalyseur dont la masse active se compose de cobalt pour 40 à 80% en poids, de 3 à 7% en poids de manganèse, de 0,1 à 3% en poids de phosphore et de 0,01 à 0,5% en poids de sodium.

**7.** Procédé selon la revendication 1, caractérisé en ce qu'on utilise un catalyseur dont la masse active se compose de cobalt pour 40% en poids au moins et contient, comme autres constituants actifs, jusqu'à 10% en poids de manganèse, jusqu'à 30% en poids de cuivre, jusqu'à 5% en poids de molybdène, jusqu'à 10% en poids de phosphore et jusqu'à 1% en poids de sodium.

**8.** Procédé selon la revendication 3, caractérisé en ce qu'on utilise un catalyseur dont la masse active se compose de cobalt pour 40 à 60% en poids, de 3 à 7% en poids de manganèse, de 0,1 à 3% en poids de phosphore, de 12 à 20% en poids de cuivre, de 0,5 à 5% en poids de molybdène et de sodium pour 0,01 à 0,5% en poids.